# EUROPÄISCHE PATENTSCHRIFT

(11) **EP 2 528 905 B1**
(45) Veröffentlichungstag und Bekanntmachung des Hinweises auf die Patenterteilung: **07.10.2015**
(21) Anmeldenummer: 11701454.8
(22) Anmeldetag: 21.01.2011
(51) Int. Cl.: C07D 311/58, C12P 17/06

(54) **VERFAHREN ZUR HERSTELLUNG VON NEBIVOLOL**
PROCESS FOR THE PREPARATION OF NEBIVOLOL
PROCÉDÉ DE PRÉPARATION DE NEBIVOLOL

(30) Priorität: 27.01.2010 DE 102010005953
(43) Veröffentlichungstag der Anmeldung: 05.12.2012
(73) Patentinhaber: Corden Pharma Colorado Inc., Boulder, CO 80301 (US)
(72) Erfinder: JAS, Gerhard, 12249 Berlin (DE); FREIFELD, Ilia, 61118 Bad Vilbel (DE); KESSELER, Kurt, 65719 Hofheim (DE)
(74) Vertreter: Wagner, Jutta
(86) Internationale Anmeldenummer: PCT/EP2011/000246
(87) Internationale Veröffentlichungsnummer: WO 2011/091968

(56) Entgegenhaltungen:
- EP-A1- 1 803 715
- WO-A2-2005/108590
- WO-A2-2008/040528
- WO-A2-2008/064827
- WO-A2-2009/121710

## Beschreibung

Die vorliegende Erfindung beschreibt ein neues Verfahren zur Herstellung von racemischen Nebivolol der allgemeinen Formel (**I**) sowie zur Synthese der reinen Enantiomeren.

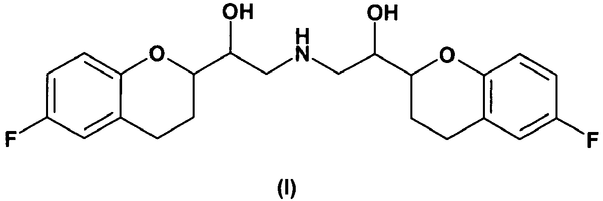

Nebivolol gehört als Wirkstoff zur Gruppe der selektiven β₁-Adreno-rezeptorenblocker (ß-Blocker) und wird zur Behandlung des Bluthochdrucks eingesetzt. Nebivolol wird als Racemat appliziert und setzt sich aus den beiden Enantiomeren [2S [2R [R [R ]]]] α,α'-[imino-bis[methylen]] bis [6-fluoro-chroman-2-methanol] (im Folgenden d-Nebivolol genannt) mit der Strukturformel **(Ia)** sowie dem [2R [2S [S [S]]]]-Enantiomer mit der Strukturformel (**Ib**) (im Folgenden I-Nebivolol genannt) zusammen.

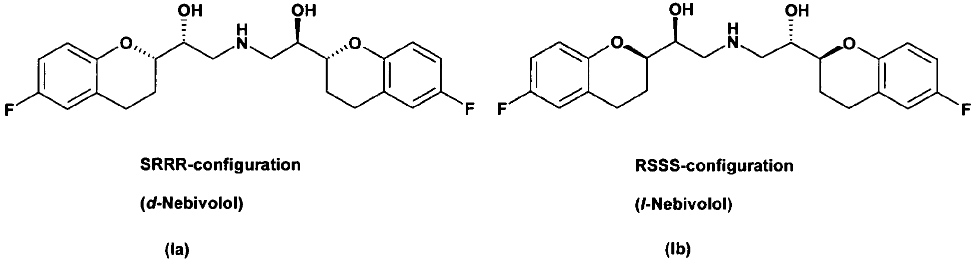

Auf Grund seiner basischen Eigenschaften kann Nebivolol in pharmazeutisch wirksame Säureadditions-Salze überführt werden, wobei das Hydrochlorid das auf dem Markt erhältliche und für die Behandlung zugelassene Salz darstellt.

Die dem Fachmann sofort ersichtliche Herausforderung in der Synthese von Nebivolol besteht in den 4 Asymmetriezentren der Zielverbindung, die theoretisch zu 16 denkbaren Enantiomeren Anlass geben. Deren Zahl wird aber auf Grund der Symmetrieeigenschaften der Verbindung auf nur 10 mögliche Enantiomere reduziert.

Es überrascht nicht, dass die Komplexität der Struktur zu einer Vielzahl von Verfahren geführt hat, die eine handhabbare Lösung propagieren. Die meisten Verfahren sind auf Grund mangelnder stereochemischer Selektion und den daraus resultierenden aufwändigen Trennungs- und Reinigungsvorgängen der zwischenzeitlich anfallenden Diastereomerengemische allerdings unpraktikabel und/oder zu teuer.

Eine nicht-stereoselektive Synthese von Nebivolol wurde erstmals von Janssen Pharmaceutica N.V. in der Anmeldung EP 0145067 (US 4,654,362) ausgehend von 6-Fluor-4-oxo-4H-1-benzopyran-2-carbonsäure beschrieben. Startpunkt ist racemischer 6-Fluor-chromancarbonsäureethylester, der über eine Reduktions-Oxidations-Sequenz in den korrespondierenden Aldehyd überführt wird. Dieser Aldehyd wird nach bekannten Verfahren in ein Gemisch von 4 stereoisomeren Epoxiden [(R,S)-, (S,R)-, (R,R)- und (S,S)-Konfiguration] überführt (Schema 1). Dieses Stereoisomerengemisch wird mittels Chromatographie in ein Gemisch der anti-konfigurierten (R,S)-, (S,R)-Epoxide bzw. syn-konfigurierten (S,S)-, (R,R)-Epoxide aufgetrennt, die die zentralen Intermediate für die weitere Synthese darstellen.

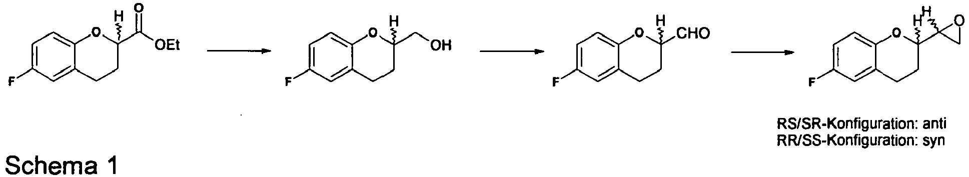

In einer Fortführung dieses Ansatzes ist in der Anmeldung EP 0334429 (US 6,545,040) eine stereoselektive Synthese von Nebivolol über die selektive Darstellung von *d*- bzw. *l*-Nebivol beschrieben. Ausgangspunkt ist die Racematspaltung der 6-Fluor-chromancarbonsäure mittels (+) Dehydroabiethylamin in die beiden Enantiomere, die dann über Standardschritte in die Epoxide überführt werden (Schema 2).

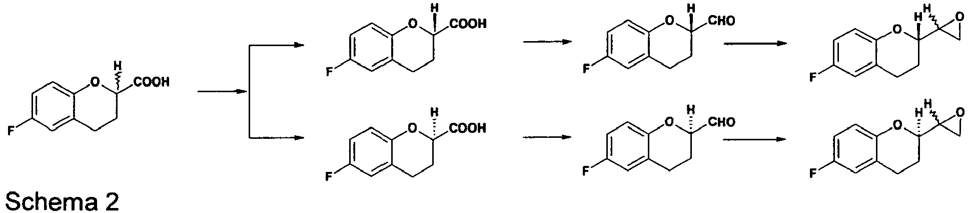

Cimex Pharma AG und Universität Zürich beschreiben in den Anmeldungen EP 1803715 und EP 1803716 Alternativverfahren zur Darstellung von racemischen Nebivolol und seiner Enantiomere. Ausgangspunkt dieser Verfahren ist die racemische Verbindung der Formel (A) mit LG = Br oder Cl, die nach einem neuartigen Verfahren hergestellt wird. Die Verbindung (A) wird durch stereoselektive Reduktion in die Alkohole der Formel (B) transformiert, aus denen ein Gemisch der Epoxide (C) generiert wird.

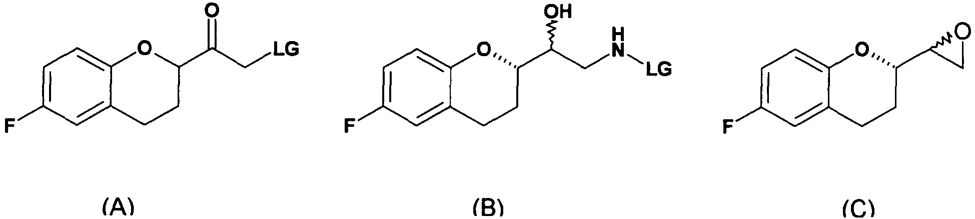

Die komplette Synthesesequenz zeigt Schema 3.

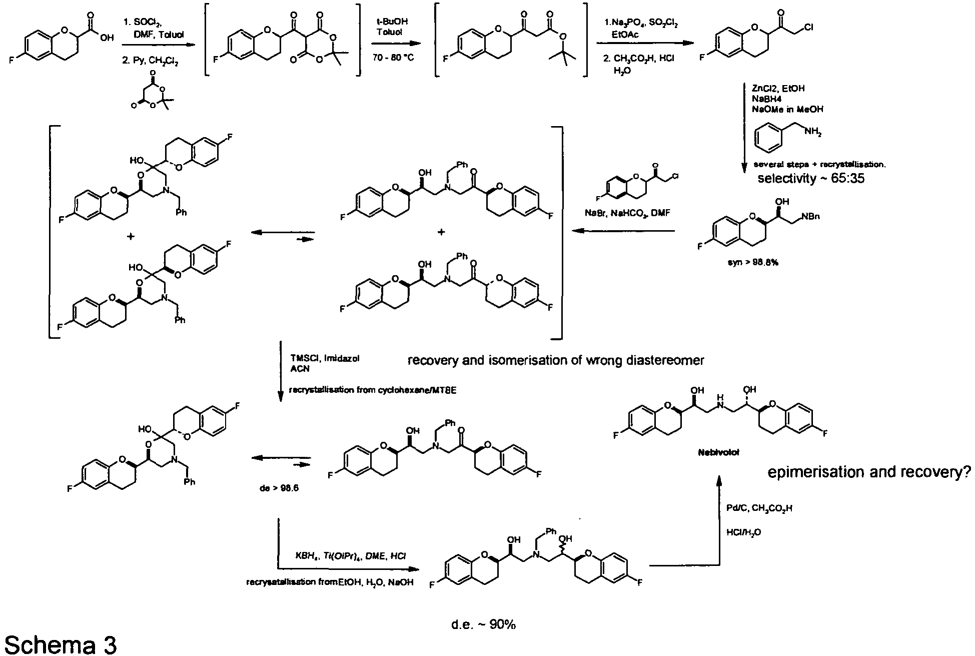

Obwohl alle Reduktionsschritte innerhalb dieses Verfahrens sehr eingehend untersucht und optimiert wurden, leidet die Gesamtsynthese unter den nur moderaten Selektivitäten, die aufwändige Kristallisations- und Rückgewinnungsschritte nötig machen.

US 2008/0221340 (Pharmacon Forschung und Beratung GmbH) und die korrespondierenden Anmeldungen EP 1919888 bzw. WO 2007/009143 beschreiben zur Darstellung von racemischen Nebivolol einen Prozess, der die Nutzung von diastereomeren Cyanhydrinen der Formel (D), die aus racemischem 6-Fluor-3,4-dihydro-2H-[1]benzopyran-2-carbaldehyd hergestellt werden, vorsieht.

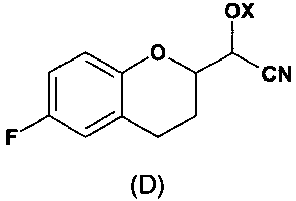

Die weitere Synthese sieht jedoch die Verwendung von Lithiumaluminiumhydrid LiALH₄ als Reduktionsmittel zur Darstellung der Amine aus den Nitrilen vor, sowie Natriumcyanoborhydrid bei einer reduktiven Aminierung. Beide Reagenzien gehören nicht zu den bevorzugten Reagenzien in der industriellen Produktion. Außerdem ist eine Reihe von fraktionierten Kristallisationsschritten mit nachfolgender Umkristallisation erforderlich, um die Diastereomere innerhalb der Synthese so aufzutrennen, dass geeignete Kupplungsvorläufer für racemisches Nebivolol zur Verfügung stehen. Es ist fraglich, ob dies zu einem kommerziell attraktiven Verfahren entwickelt werden kann.

Die kürzlich offengelegte Anmeldung WO 2009/121710 (ZACH SYSTEM S.P.A.) beschreibt einen Prozess zur Darstellung von Nebivolol, der ein Haloketon der Formel (E) nutzt, dass mittels (+) oder (-) B-Chlordiisopinacampheylboran (DIP-Chlorid) stereoselektiv zu diastereomeren Alkoholen der Formel (F) reduziert wird, die als Intermediate für die Herstellung von Nebivolol fungieren.

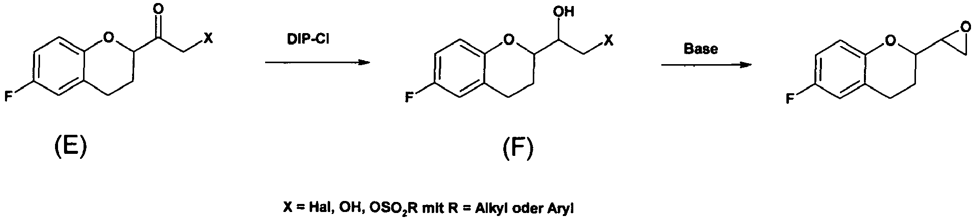

Es wird berichtet, dass die Reduktion mittels DIP-CI mit sehr guter Diastereoselektivität (ca. 99%) erfolgt. Allerdings führt auch dieses Konzept zur Ausbildung von Diastereomerengemischen bei (F), die eine spätere Auftrennung erfordern wie z.B. über eine hydrolytische kinetische Racematspaltung, wie sie in der Anmeldung WO 2008/040528 beschrieben ist.

Weitere Verfahren zur Darstellung von Nebivolol werden beansprucht in den Anmeldungen WO 2006/025070 (Torrent Pharmaceuticals), WO 2006/016376 und WO 2007/083318 (Hetero Drugs). Auch in diesen Anmeldungen sind aber lediglich Diastereomerengemische von Intermediaten beschrieben, die letztlich chromatographischen Trennungen erfordern.

WO 2004/041805 (EGIS GYOGYSZERGYAR) beschreibt die Synthese von 4 enantiomerenreinen Intermediaten, die durch geeignete Kopplungen in zwei enantiomerenreine Vorstufen von *d*- bzw- *l*-Nebivolol überführt werden. Weitere chemische Manipulation einer racemischen Mischung dieser Vorstufen erbringt abschließend racemisches Nebivolol. Durch die Verwendung von Derivaten des D- bzw. L-Glycerinaldehyds aus dem chiral pool wird mit der separaten Darstellung der diasteromeren Intermediate (G a,b) die Basis gelegt für die spätere Darstellung der 4 enantiomerenreinen Epoxide (H a-d) über Standardmanipulationen wie Hydrolyse des Acetals gefolgt von Tosylierung der primären Hydroxylfunktion sowie abschließender Zyklisierung zu den erwähnten Epoxiden (Schema 4). Die dabei erforderliche Auftrennung der anfänglich erhaltenen Diastereomerengemische behaupten die Anwender allein durch selektive Kristallisationsverfahren zu erreichen.

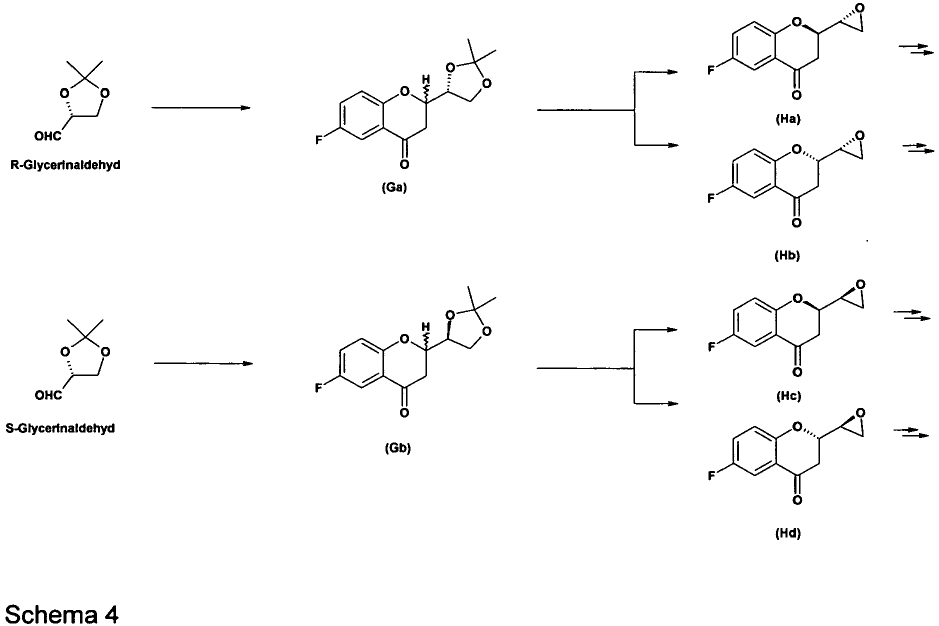

In der Literatur finden sich einige Vorschläge (z.B. S. Chandrasekhar, V. Reddy: Tetrahedron 56 (2000), 6339-6344; C.W: Johannes, M.S: Visser, G.S: Weatherhead, A.J. Hoveyda: J. Am. Chem. Soc. 120 (1998), 8340-8347), die sich mit der Synthese von reinen Enantiomeren des Nebivolols beschäftigen. Eine Übertragung dieser Synthesen auf den industriellen Maßstab ist jedoch aus Kostengründen kaum möglich.

Aufgabe der vorliegenden Erfindung ist daher eine einfachere und/oder kostengünstigere Herstellung von racemischem Nebivolol.

Überraschend wird diese Aufgabe durch die unabhängige Synthese von *d*- bzw. *l*-Nebivolol (**Ia**) bzw. (**Ib**) wie in Schema 5 skizziert gelöst. Enantiomerenreines Nebivolol (**Ia**) bzw. (**Ib**) wird dabei über die individuelle Kupplung der 4 separaten, enantiomerenreinen Intermediate (**IIa-d**) mit den enantiomerenreinen Vorläufern der Formel (**IIIa-d**) erhalten. Die Herstellung dieser Voräufer (**IIIa-d**) erfolgt über die beiden enantiomerenreinen Ketonderivate (**IVa**) und (**IVb**). Die Enantiomeren werden dann zu racemischem Nebivolol gemischt.

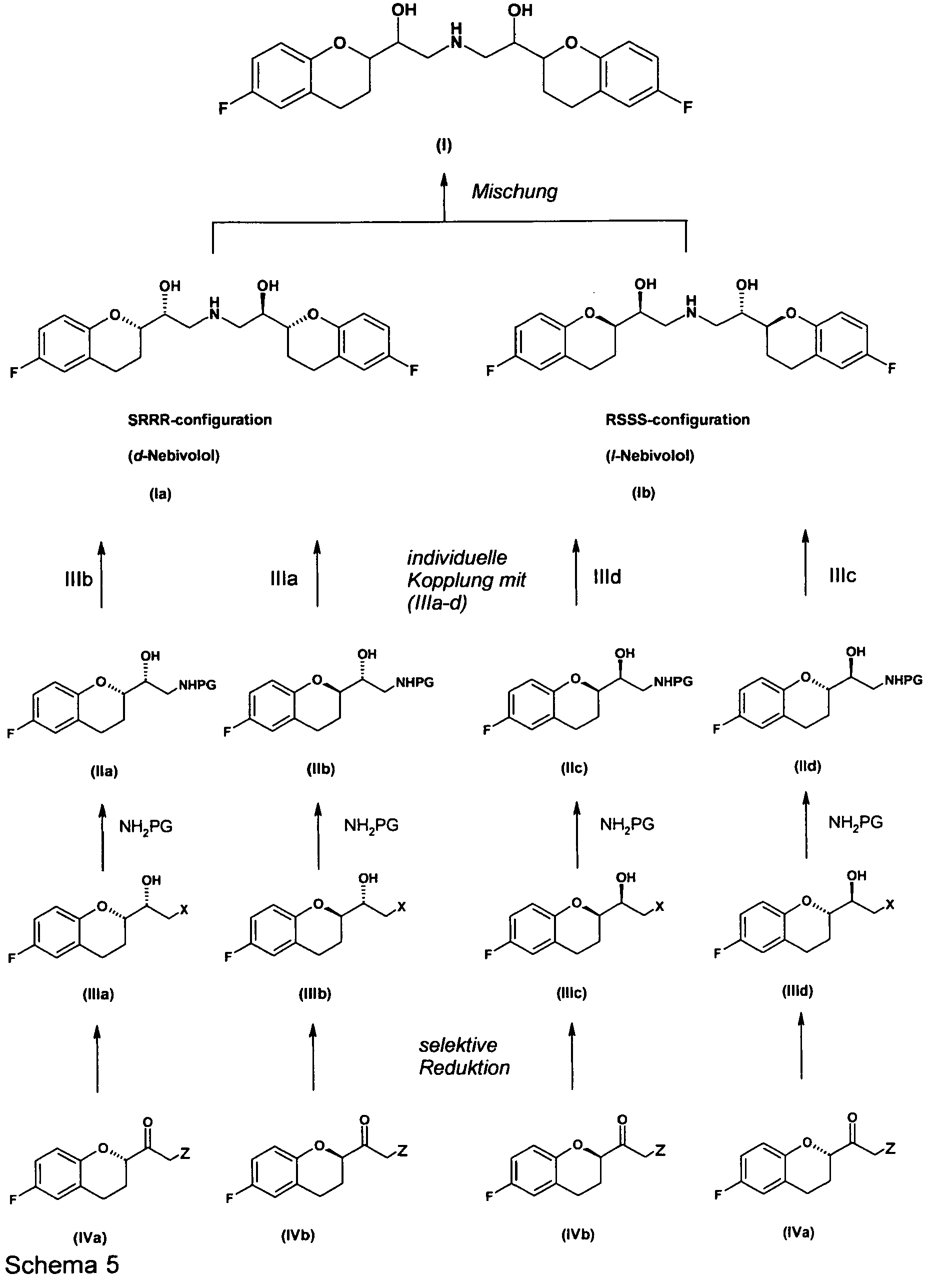

Mit PG ist in (**IIa-d**) jeweils ein Wasserstoffatom oder eine Aminschutzgruppe bezeichnet. Die Gruppe X in (**IIIa-d**) steht für ein Halogenatom, eine Hydroxylfunktion, eine Acylgruppe, eine Alkylsulfonyloxygruppe oder eine Arylsulfonyloxygruppe. Die Gruppe Z in (**IVa,b**) steht ebenfalls für ein Halogenatom, eine Hydroxylfunktion, eine Acylgruppe, eine Alkylsulfonyloxygruppe oder eine Arylsulfonyloxygruppe.

Die Verbindungen (**IIa-d**) und (**IIIa-d**) stellen die Schlüsselintermediate für die Synthese von racemischem Nebivolol dar. Ein weiteres Ziel der vorliegenden Erfindung ist es, für die Herstellung der Intermediate (**IIa-d**) und deren Vorläufer (**IIIa-d**) Verfahren bereitzustellen, die die selektive Synthese dieser individuellen Isomeren mit hohen chemischen Reinheiten und vor allem hohen optischen Reinheiten (>98%) erlauben. Unter dieser Voraussetzung kann auf zwischenzeitliche Aufreinigungsschritte verzichtet werden. Notwendig ist ggfs nur eine Umkristallisation der Verbindungen der Formel (**Ia**) bzw. (**Ib**), wodurch ein äußerst effizienter und ökonomischer Prozess für die Herstellung von Nebivolol gegeben ist.

Entsprechend der Struktur des Targetmoleküls kann Nebivolol sehr effizient und selektiv aus enantiomerenreinen Chromanketonen IV mit geeigneter Abgangsgruppe, z.B. Z = Hydroxy, Chlor, Brom, Iod, Mesylat oder Tosylat, dargestellt werden.

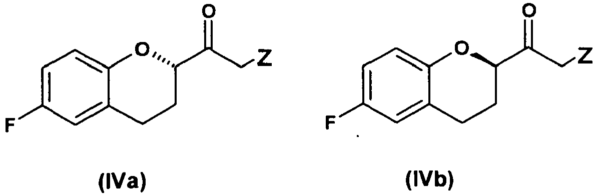

Die stereoselektive Reduktion dieser Ketone erbringt die korrespondierenden diastereomeren Alkohole (**IIIa-d**), die Vorläufer für Nebivolol sind. Voraussetzung ist, dass die diastereomeren Alkohole selektiv in sehr hoher Reinheit gewonnen werden können. Die Alkohole (**IIIa-d**) werden erfindungsgemäß ohne Aufreinigung aus den Verbindungen der Formel (**IVa,b**) mit Diastereomerenüberschüssen von > 98%, bevorzugt mit einem Diastereomerenüberschuss > 99%, besonders bevorzugt mit einem Diastereomerenüberschuss > 99,5%, erhalten.

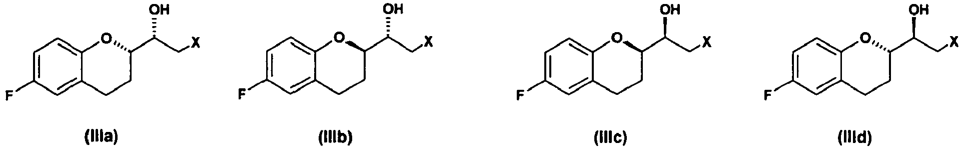

Als Reduktionsmittel kommen chirale komplexe Hydride, chirale Borverbindungen (z.B. das von Corey entwickelte chirale Boran CBS), chirale Katalysatoren für Transferhydrierungen und katalytische Hydrierungen sowie Enzyme in Betracht.

Überraschend wurde gefunden, dass eine sehr stereoselektive Reduktion der Chromanketone (**IVa**) und (**IVb**) mit Enzymen möglich ist. Enzymatische Prozesse zur Darstellung der enantiomerenreinen Alkohole (III a-d) sind bislang nicht beschrieben. Die selektive, enzymatische Reduktion von Ketonen zu chiralen sekundären Alkoholen bei der Synthese von chiralen Bausteinen ist an sich bekannt und kann mittels Alkoholdehydrogenasen und Ketoreduktasen (jeweils mit und ohne Cofaktor-Regenierung) durchgeführt werden. Diese enzymatischen Verfahren eignen sich sehr gut auch für industrielle Synthesen, da in der Regel sehr hohe Umsätze erreicht werden können bei gleichzeitig sehr hoher Selektivität. Insbesondere ist die enzymatische Reduktion von α-Chlorketonen eingehend untersucht worden. Die resultierenden Halohydrine können entweder direkt oder über den Umweg über die korrespondierenden Epoxide mit Aminen gekuppelt werden. Angewendet auf Nebivolol bedeutet dies, dass die Zielsubstanz eindeutig auf Basis der enantiomerenreinen Intermediate dargestellt werden kann.

Bei einem enzymatischen Screening von kommerziell erhältlichen oder bei der Anmelderin verfügbaren Alkoholdehydrogenasen wurde gefunden, dass die Vorläuferalkohole (**IIIa-d**) enzymatisch auf mehreren Wegen in hoher Selektivität gebildet werden können. Viele Alkoholdehydrogenasen verfügen über eine ausgewiesene R- oder S Selektivität hinsichtlich des neu gebildeten Asymmetriezentrums.

Mittels enzymatischer Reduktion lassen sich erfindungsgemäß die Chromanketone der Formel **(IV a**, **b**) und in quantitativer Ausbeute mit einer Diastereomeren- bzw. Enantiomerenreinheit von jeweils > 99 % gezielt in die Alkohole der Formeln III a-d überführen. Vorzugsweise steht X für Chlor.

Die vorliegende Erfindung betrifft daher auch ein Verfahren zur enzymatischen Reduktion von Chromanketonen der Formel (IV), mit X = Halogenatom, Hydroxylgruppe oder daraus gewonnene Alkyl- oder Arylsulfonyloxy-Gruppen, zu den enantiomerenreinen Verbindungen der allgemeinen Formel (III), 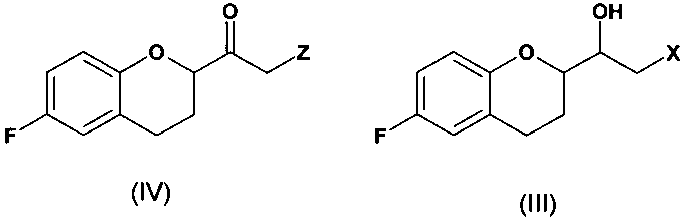 wobei Z entweder ein Halogenatom darstellt, eine Hydroxylgruppe oder daraus gewonnene Alkyl- oder Arylsulfonyloxy-Gruppen. Verbindungen der allgemeinen Formel (IV) können in racemischer oder enantiomerenreiner Form leicht nach dem Stand der Technik hergestellt werden.

Generell können die enzymatischen Reaktionen in wässrigen Pufferlösungen bei Raumtemperatur durchgeführt werden. Als Enzyme können Alkoholdehydrogenasen oder Ketoreduktasen eingesetzt werden, wobei die Reduktion optional mit Cofaktoregenerierung durchgeführt werden kann. Die Enzyme können dabei in isolierter oder immobilisierter Form eingesetzt werden oder aber über rekombinante Ganzzellsysteme zur Verfügung gestellt werden.

Die enzymatischen Reduktionen werden mittels in-process-Kontrollen so gefahren, dass die Stereoselektivitäten möglichst hoch sind, ggfs. durch vorzeitigen Abbruch der Reaktion bei unvollständigem Umsatz. Zielgröße und erfindungsgemäß erreichbar ist dabei ein ee-Wert von > 99% des gewünschten Enantiomeren.

Nach Abtrennung des Enzyms bzw. der Zellen durch Filtration oder Zentrifugation wird die wässrige Lösung mit organischen Lösungsmitteln extrahiert. Bevorzugt sind dabei mit Wasser nicht mischbare Lösungsmittel wie Methyltertiärbutylether oder Ethylacetat. Nach Trocknen der organischen Phasen erbringt das Abdestillieren des Lösungsmittels die enantiomerenreinen Alkohole als Rohprodukt in so hoher Reinheit, dass auf weitere Aufreinigung verzichtet werden kann und die Produkte direkt weiter umgesetzt werden können.

Weiter betrifft die vorliegende Erfindung die Bereitstellung eines Verfahrens zu direkten Umwandlung der chiralen Alkohole (III) zu den enantiomerenreinen Verbindungen der Formel (II) 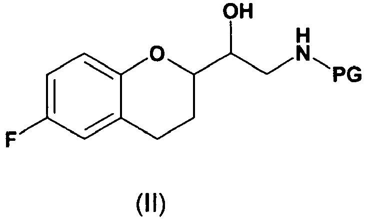 wobei PG entweder ein H-Atom oder eine Schutzgruppe (bevorzugt Benzyl) bedeutet. Die Intermediate der Formel (**IIa-d**) werden ohne Aufreinigung aus den Verbindungen der Formel (**IIIa-d**) mit Enantiomerenüberschüssen von > 98%, bevorzugt mit einem Enantiomerenüberschuss > 99%, besonders bevorzugt mit einem Enantiomerenüberschuss > 99,5%, erhalten. Die Umsetzung erfolgt z.B. entweder durch direkte Kupplung mit dem Amin oder über den Umweg der korrespondierenden Epoxide in aprotischen organischen Lösungsmitteln wie z.B. THF oder Dioxan unter Rückflussbedingungen. Bevorzugt erfolgt eine direkte Umsetzung mit Ammoniak oder einem Arylamin, insbesondere mit Benzylamin, die Ausbeuten liegen dabei um 85-90 % ohne Verlust an Diastereomeren- oder Enantiomerenreinheit. Nicht umgesetzte Edukte lassen sich durch übliche Extraktionsprozesse wie Waschen der kristallinen Rohprodukte aus dem Rohprodukt entfernen. Eine Aufreinigung der Rohprodukte erwies sich als nicht erforderlich.

Weiter betrifft die vorliegende Erfindung die Überführung der Verbindungen der Formel **(II)** in d- bzw. I-Nebivolol. Dazu werden die Aminoalkohole (IIa-d) mit den Alkoholen (IIIa-d) unter Einwirkung einer anorganischen Base wie Kaliumcarbonat in einem aprotischen Lösungsmittel wie THF oder Dioxan über Kreuz gekuppelt, und zwar (**IIa**) mit (**IIIb**) oder (**IIb**) mit (**IIIa**) sowie (**IIc**) mit (**IIId**) oder (**IId**) mit (**IIIc**). Gegebenenfalls wird eine am Stickstoff vorhandene Schutzgruppe entfernt, beispielsweise eine Benzylgruppe durch Hydrierung mittels 5 % Pd/C in einem Solvensgemisch z.B. aus Ethanol/Eisessig typischerweise bei ca. 50°C und einem Druck von ca. 3bar. D-Nebivolol (**Ia**) und I-Nebivolol (**Ib**) werden ohne vorherige Aufreinigung in Enantiomerenüberschüssen von > 98%, bevorzugt mit einem Enantiomerenüberschuss > 99%, besonders bevorzugt mit einem Enantiomerenüberschuss > 99,5%, erhalten. Racemisches Nebivolol wird durch Mischen von d-Nebivolol (**Ia**) und I-Nebivolol (**Ib**) erhalten.

### Beispiele

Die folgenden Beispiele veranschaulichen die Erfindung, ohne sie jedoch auf die speziell beschriebenen Bedingungen und Substanzen zu beschränken.

Herstellung der Pufferlösung für die enzymatische Reduktion: Triethanolamin (4g; 26,5mmol) wird in Wasser (215ml) gelöst. Unter Rühren wird die Lösung mit 36% HCl (2,3g) auf pH6,99 eingestellt. Man gibt ZnCl₂ (0,057g) und füllt auf 270ml auf. Anschließend wird Glycerin (37,5g) dazugegeben und gut durchmischt.

Durchführung der enzymatischen Reduktion: In einem Kolben wird Isopropanol (20g) vorgelegt und unter Eiskühlung auf 0-5°C gekühlt. Man versetzt mit ß-NAD (10mg) und gibt vorgekühlte Pufferlösung (10ml) hinzu. Anschließend werden bei 0°C 50mmol des Chlorketons in die Reaktionsmischung gegegeben und abschließend mit 6000 Einheiten (S)- oder (R)-selektiver Alkoholdehydrogenase versetzt. Man erwärmt auf 20-25°C und lässt 24h rühren. Nach vollständiger Umsetzung wird die Reaktionslösung zentrifugiert und nach Phasentrennung mit Ethylacetat (2x 10ml extrahiert). Die organischen Phasen werden mit ges. NaCl-Lösung (20ml) gewaschen und anschließend über Na₂SO₄ getrocknet. Abdestillieren des Lösungsmittels im Vakuum erbringt das Rohprodukt.

### Beispiel 1:

Herstellung von (*R*)-2-chloro-1-((*S*)-6-fluor-3,4-dihydro-2H-chromen-2-yl)-ethanol 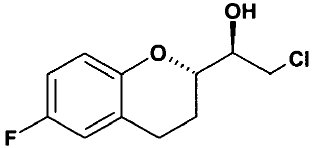 Entsprechend der obigen Angaben erhielt man aus 1-(2*S*)-6-fluorchroman-2-yl-2-chlorethan-1-on und (*R*)-selektiver Alkoholdehydrogenase 11,42 g (99%.d.Th.) (*R*)-2-chloro-1-(*S*)-6-fluor-3,4-dihydro-2H-chromen-2-yl)-ethanol (d.e. 99,7%).
LC-MS: m/z = 230,232 (MH⁺, 100%)

### Beispiel 2:

Herstellung von (*R*)-2-chloro-1-((*R*)-6-fluor-3,4-dihydro-2H-chromen-2-yl)-ethanol 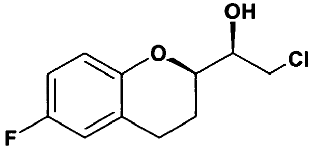 In analoger Weise zu Beispiel 1 erhält man aus 1-(2*R*)-6-fluorchroman-2-yl-2-chlorethan-1-on und (*R*)-selektiver Alkoholdehydrogenase 11,07 g (96%.d.Th.) (*R*)-2-chloro-1-((*R*)-6-fluor-3,4-dihydro-2H-chromen-2-yl)-ethanol (d.e. 99,4%). LC-MS: m/z = 230,232 (MH⁺, 100%)

### Beispiel 3:

Herstellung von (*S*)-2-ch)oro-1-((*R*)-6-fluor-3,4-dihydro-2H-chromen-2-yl)-ethanol

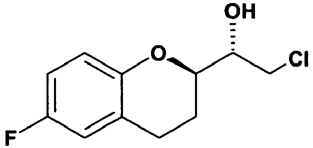

In analoger Weise zu Beispiel 1 erhielt man aus 1-(2*R*)-6-fluorchroman-2-yl-2-chlorethan-1-on unter Verwendung (*S*)-selektiver Alkoholdehydrogenase 11,42 g (99%.d.Th.) (*S*)-2-chloro-1-((*R*)-6-fluor-3,4-dihydro-2H-chromen-2-yl)-ethanol (d.e. 99,8%).
LC-MS: m/z = 230,232 (MH^{+,} 100%)

### Beispiel 4:

Herstellung von (*S*)-2-chloro-1-((*S*)-6-fluor-3,4-dihydro-2H-chromen-2-yl)-ethanol

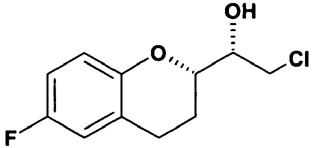

In analoger Weise zu Beispiel 1 erhielt man aus 1-(2S)-6-fluorchroman-2-yl-2-chlorethan-1-on unter Verwendung (*S*)-selektiver Alkoholdehydrogenase 10,72 g (93%.d.Th.) (*S*)-2-chloro-1-((*S*)-6-fluor-3,4-dihydro-2H-chromen-2-yl)-ethanol (d.e. 99,5%).
LC-MS: m/z = 230,232 (MH^{+,} 100%)

### Beispiel 5:

Herstellung von (*S*)-2-benzylamino-1-((*S*)-6-fluor-3,4-dihydro-2H-chromen-2-yl)-ethanol

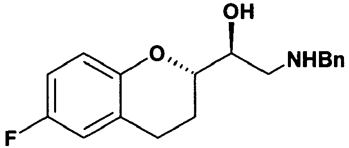

Zu einer Lösung von (*R*)-2-chloro-1-((*S*)-6-fluor-3,4-dihydro-2H-chromen-2-yl)-ethanol (10 mmol, 2,307g) in THF (100ml) wurde Benzylamin (22 mmol, 2,14g) gegeben und die resultierende Mischung 20h unter Rückfluss erhitzt. Anschließend wurde das Lösungsmittel im Vakuum abdestilliert. Der verbleibende Rückstand wurde mit tert. Butylmethylether (40ml) dispergiert und der resultierende Feststoff anschließend abgesaugt. Nach Waschen mit MTBE (20ml) und Trocknung im Vakuum erhielt man (*S*)-2-benzylamino-1-((*S*)-6-fluor-3,4-dihydro-2H-chromen-2-yl)-ethanol (2,74g, 91% d.Th.) als schwach gelbes Pulver in einer HPLC-Reinheit von 98,5% und einem e.e -Wert von 99,6%.
LC-MS: m/z = 302 (MH^{+,} 100%)

### Beispiel 6:

Herstellung von (*S*)-2-benzylamino-1-((*R*)-6-fluor-3,4-dihydro-2H-chromen-2-yl)-ethanol 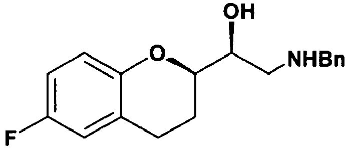 Entsprechend der Vorschrift in Beipiel 5 erhielt man aus (*R*)-2-chloro-1-((*R*)-6-fluor-3,4-dihydro-2H-chromen-2-yl)-ethanol (10 mMol, 2,307g) durch Reaktion mit Benzylamin (*S*)-2-benzylamino-1-((*R*)-6-fluor-3,4-dihydro-2H-chromen-2-yl)-ethanol (2,56g, 85% d.Th.) mit einer HPLC-Reinheit von 99,2% und einem e.e-Wert von 99,4%.
LC-MS: m/z = 302 (MH^{+,} 100%)

### Beispiel 7:

Herstellung von (*R*)-2-benzylamino-1-((*R*)-6-fluor-3,4-dihydro-2H-chromen-2-yl)-ethanol

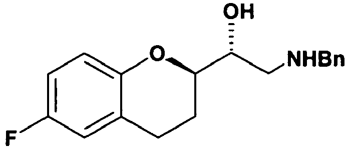

Entsprechend der Vorschrift in Beipiel 5 erhielt man aus (*S*)-2-chloro-1-((*R*)-6-fluor-3,4-dihydro-2H-chromen-2-yl)-ethanol (10 mMol, 2,307g) durch Reaktion mit Benzylamin (*R*)-2-benzylamino-1-((*R*)-6-fluor-3,4-dihydro-2H-chromen-2-yl)-ethanol (2,47g, 82% d.Th.) mit einer HPLC-Reinheit von 98,8% und einem e.e-Wert von 99,7%.
LC-MS: m/z = 302 (MH^{+,} 100%)

### Beispiel 8:

Herstellung von (R)-2-benzylamino-1-((S)-6-fluor-3,4-dihydro-2H-chromen-2-yl)-ethanol

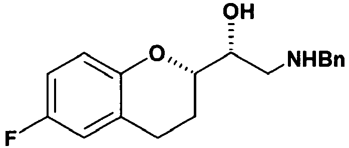

Entsprechend der Vorschrift in Beipiel 5 erhielt man aus (*S*)-2-chloro-1-((*S*)-6-fluor-3,4-dihydro-2H-chromen-2-yl)-ethanol (10 mMol, 2,307g) durch Reaktion mit Benzylamin (*R*)-2-benzylamino-1-((*S*)-6-fluor-3,4-dihydro-2H-chromen-2-yl)-ethanol (2,80g, 93% d.Th.) mit einer HPLC-Reinheit von 98,6% und einem e.e-Wert von 99,5%.
LC-MS: m/z = 302 (MH^{+,} 100%)

### Beispiel 9: Herstellung von benzyliertem d-Nebivolol

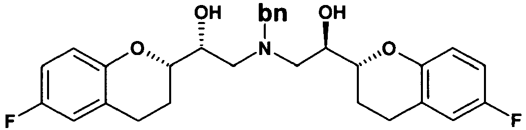

Zu einer Lösung von (*R*)-2-benzylamino-1-((*S*)-6-fluor-3,4-dihydro-2H-chromen-2-yl)-ethanol (10 mMol, 3,014g) in Dioxan (50ml) gab man (*S*)-2-chloro-1-((*R*)-6-fluor-3,4-dihydro-2H-chromen-2-yl)-ethanol (10mMol, 2,31g) und K₂CO₃ (10mMol). Die Mischung wurde 24h unter Rückfluss erhitzt. Nach Heißfiltration wurde das Lösungsmittel im Vakuum abdestilliert und der Rückstand mit Ethylacetat (20ml) digeriert. Der Feststoff wurde abfiltriert und gründlich mit Ethylacetat gewaschen. Nach dem Trocknen im Vakuum erhielt man 3,62g Produkt (73% d.Th.) in einer HPLC-Reinheit von 98,6% und einem e.e.-Wert von 99,6%.
LC-MS: m/z = 496 (MH^{+,} 100%)

### Beispiel 10: Herstellung von benzyliertem l-Nebivolol

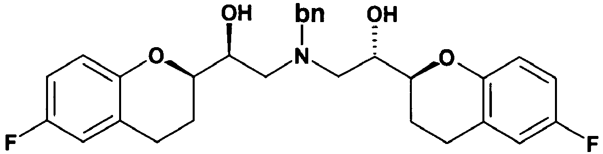

In analoger Weise zu Beispiel 9 erhielt man aus (*S*)-2-benzylamino-1-((*R*)-6-fluor-3,4-dihydro-2H-chromen-2-yl)-ethanol (10mMol, 3,014g) und (R)-2-chloro-1-((*S*)-6-fluor-3,4-dihydro-2H-chromen-2-yl)-ethanol (10 mMol, 2,307g) das Produkt (3,42g, 73% d.Th.) in einer HPLC-Reinheit von 99,1% und einem e.e.-Wert von 99,5%.
LC-MS: m/z = 496 (MH^{+,} 100%)

### Beispiel 11: Herstellung von d-Nebivolol

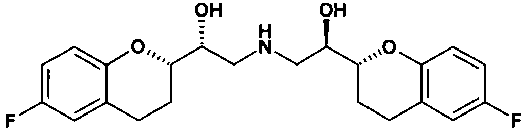

Benzyliertes *d*-Nebivolol aus Beispiel 9 (4,96g; 10mmol) wurde in Ethanol (50ml) und Eisessig (5ml) mit 5% Pd/C bei einem Druck von 2-3 bar und einer Temperatur von 40-50°C debenzyliert. Die Reaktionsmischung wurde über Celite filtriert und das Filtrat im Vakuum eingeengt. Spuren an Eisessig wurden durch nachfolgende Evaporation mit 2x 25ml Ethanol beseitigt. Das erhaltene Rohmaterial wurde mit Ethylacetat (50ml) versetzt, filtriert und anschließend zum Rückfluss erhitzt. Nach Abkühlen und Abfiltrieren erhielt man *d*-Nebivolol (3,98g; 98%d.Th.) in einer chemischen Reinheit von 99,5% und einem Enantiomerenüberschuss von 99,6%
LC-MS: m/z = 406 (MH⁺, 100%)

### Beispiel 12: Herstellung von l-Nebivolol

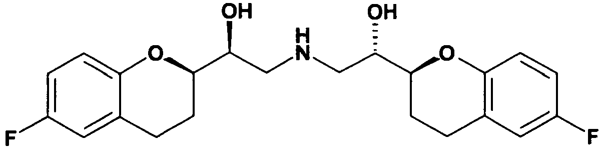

In analoger Weise zu Beispiel 11 erhielt man aus 4,96g (10mmol) benzyliertem *l-*Nebivolol aus Beispiel 10 l-Nebivolol (3,86g; 95% d.Th.) in einer chemischen Reinheit von 99,4% und einem Enantiomerenüberschuss von 99,5%.
LC-MS: m/z = 406 (MH⁺, 100%)

## Patentansprüche

1. Verfahren zur Synthese von racemischem Nebivolol der Formel (**I**) 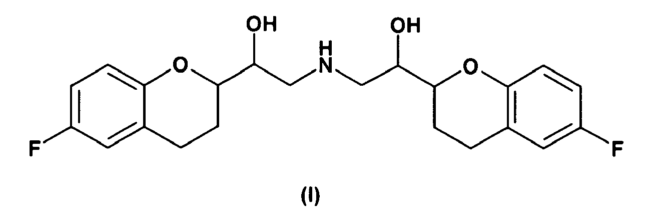 aus den enantiomerenreinen Verbindungen der Formel IVa und IVb 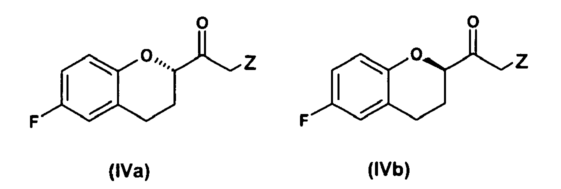 wobei racemisches Nebivolol durch Mischen der enantiomerenreinen Verbindungen d-Nebivolol (Ia) und I-Nebivolol (Ib) erhalten wird, 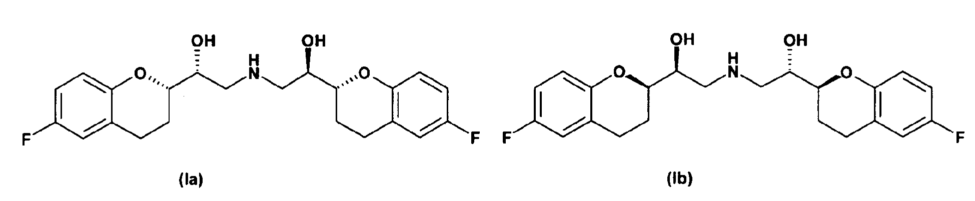 die Verbindungen d-Nebivolol (**Ia**) und I-Nebivolol (**Ib**) unabhängig voneinander als enantiomerenreine Verbindungen synthetisiert werden, indem (**Ia**) und (**Ib**) jeweils durch individuelle Kopplungen der 4 enantiomerenreinen Intermediate der Formel (**IIa-d**) mit den korrespondierenden Vorläufern der Formel (**IIIa-d**) hergestellt werden, 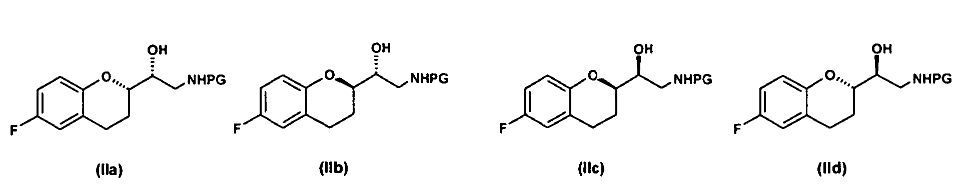 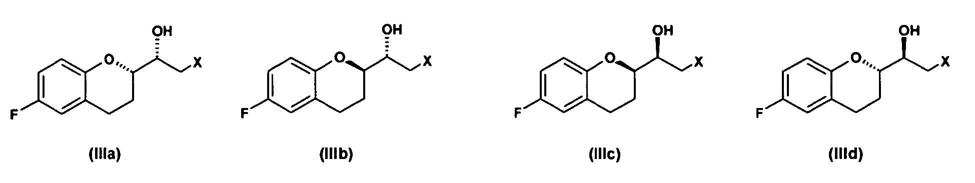 wobei durch Kopplung von (**IIa**) mit (**IIIb**) oder (**IIb**) mit (**IIIa**) d-Nebivolol (**Ia**) und durch Kopplung von (**IIc**) mit (**IIId**) oder (**IId**) mit (**IIIc**) I-Nebivolol (**Ib**) gewonnen wird und PG in den Intermediaten der Formel (**IIa-d**) ein Wasserstoffatom oder eine Aminschutzgruppe bedeutet und X in den Vorläufern der Formel (**IIIa-d**) ein Halogenatom, eine Hydroxylgruppe, eine Acylgruppe, eine Alkylsulfonyloxygrupe oder eine Arylsulfonyloxygruppe bedeutet,
die Intermediate der Formel (**IIa-d**) individuell aus den jeweiligen direkten Vorläufern der Formel (**IIIa-d**) gebildet werden, wobei Intermediat (**IIa**) aus (**IIIa**), Intermediat (**IIb**) aus (**IIIb**), Intermediat (**IIc**) aus (**IIIc**) und Intermediat (**IId**) aus (**IIId**) gebildet wird,
die Vorläufer der Formel (**IIIa-d**) spezifisch durch Reduktion der enatiomerenreinen Ketonvorstufen der Formel (**IVa,b**) gebildet werden, wobei die Vorläufer der Formel (**IIIa**) und (**IIId**) aus der Ketonvorstufe der Formel (**IVa**), und die Vorläufer der Formel (**IIIb**) und (**IIIc**) aus der Ketonvorstufe der Formel (**IVb**) hervorgehen und Z in den Ketonvorstufen (**IVa,b**) ein Halogenatom, eine Hydroxylfunktion, eine Acylgruppe, eine Alkylsulfonyloxygruppe oder eine Arylsulfonyloxygruppe bedeutet.

2. Verfahren gemäß Anspruch 1, **dadurch gekennzeichnet, dass** die Vorläufer (**IIIa-d**) durch diastereoselektive Reduktion mit einem hochselektiven, chiralen Reduktionsmittel aus den enantiomerenreinen Ketonvorstufen (**IV a**, **b**) gebildet werden.

3. Verfahren gemäß Anspruch 2, **dadurch gekennzeichnet, dass** für die Reduktion der Verbindungen (**IVa,b**) ein Reduktionsmittel ausgewählt unter chiralen komplexen Hydriden, chiralen Borverbindungen, chiralen Katalysatoren für Transferhydrierungen und katalytische Hydrierungen und Enzymen eingesetzt wird.

4. Verfahren gemäß Anspruch 3, **dadurch gekennzeichnet, dass** für die Reduktion der Verbindungen (**IVa,b**) Enzyme eingesetzt werden.

5. Verfahren gemäß Anspruch 4, **dadurch gekennzeichnet, dass** für die Reduktionen der Verbindungen (**IVa,b**) Alkoholdehydrogenasen und Ketoreduktasen mit oder ohne Co-Faktorregenerierung eingesetzt werden

6. Verfahren gemäß mindestens einem der Ansprüche 1 bis 5, **dadurch gekennzeichnet, dass** die Intermediate der Formel (**IIa-d**) aus den Vorläufern der Formel (**IIIa-d**) durch Umsetzung mit Ammoniak oder einem Arylamin, vorzugsweise Benzylamin, gebildet werden.

7. Verfahren zur Synthese von Enantiomeren des Nebivolol der Formel (**I**) 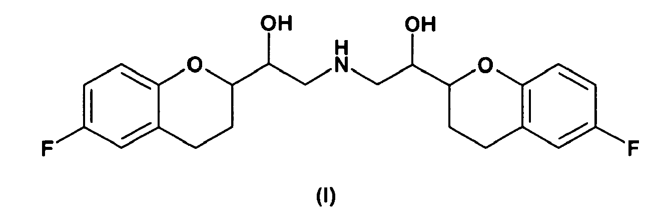 aus den enantiomerenreinen Verbindungen der Formel IVa und IVb 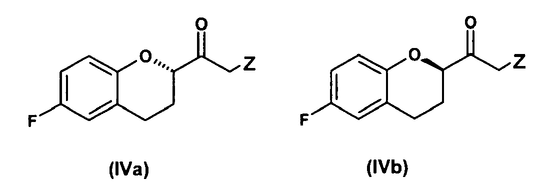 wobei die Enantiomere des Nebivolol durch individuelle Kopplungen der 4 enantiomerenreinen Intermediate der Formel (**IIa-d**) mit den korrespondierenden Vorläufern der Formel (**IIIa-d**) synthetisiert werden, 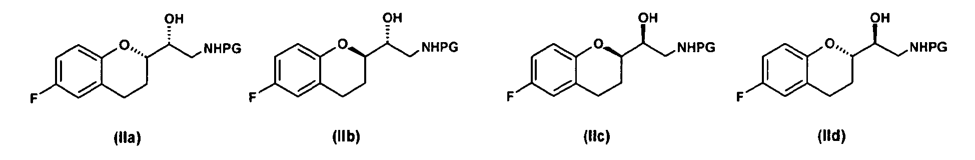 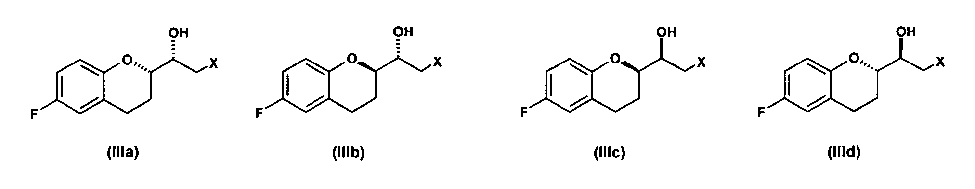 wobei PG in den Intermediaten der Formel (**IIa-d**) ein Wasserstoffatom oder eine Aminschutzgruppe bedeutet und X in den Vorläufern der Formel (**IIIa-d**) ein Halogenatom, eine Hydroxylgruppe, eine Acylgruppe, eine Alkylsulfonyloxygrupe oder eine Arylsulfonyloxygruppe bedeutet,
die Intermediate der Formel (**IIa-d**) individuell aus den jeweiligen direkten Vorläufern der Formel (**IIIa-d**) gebildet werden, wobei Intermediat (**IIa**) aus (**IIIa**), Intermediat (**IIb**) aus (**IIIb**), Intermediat (**IIc**) aus (**IIIc**) und Intermediat (**IId**) aus (**IIId**) gebildet wird,
die Vorläufer der Formel (**IIIa-d**) spezifisch durch Reduktion der enantiomerenreinen Ketonvorstufen der Formel (**IVa,b**) gebildet werden, wobei die Vorläufer der Formel (**IIIa**) und (**IIId**) aus der Ketonvorstufe der Formel (**IVa**), und die Vorläufer der Formel (**IIIb**) und (**IIIc**) aus der Ketonvorstufe der Formel (**IVb**) hervorgehen und Z in den Ketonvorstufen (**IVa,b**) ein Halogenatom, eine Hydroxylfunktion, eine Acylgruppe, eine Alkylsulfonyloxygruppe oder eine Arylsulfonyloxygruppe bedeutet.

8. Verfahren zur Synthese von racemischem Nebivolol der Formel (**I**) 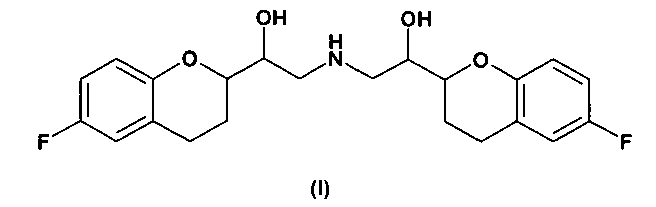 wobei racemisches Nebivolol durch Mischen der enantiomerenreinen Verbindungen d-Nebivolol (**Ia**) und I-Nebivolol (**Ib**) erhalten wird, 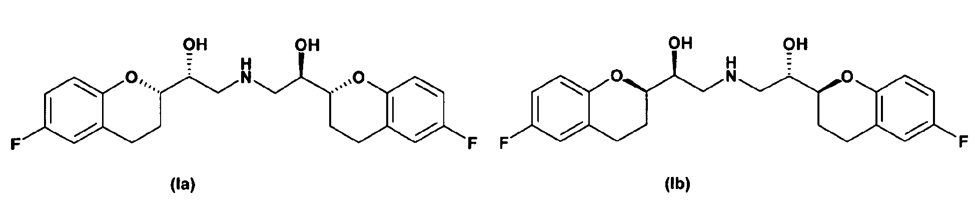 die Verbindungen d-Nebivolol (**Ia**) und I-Nebivolol (**Ib**) unabhängig voneinander als enantiomerenreine Verbindungen synthetisiert werden, indem (**Ia**) und (**Ib**) jeweils durch individuelle Kopplungen der 4 enantiomerenreinen Intermediate der Formel (**IIa-d**) mit den korrespondierenden Vorläufern der Formel (**IIIa-d**) hergestellt werden, 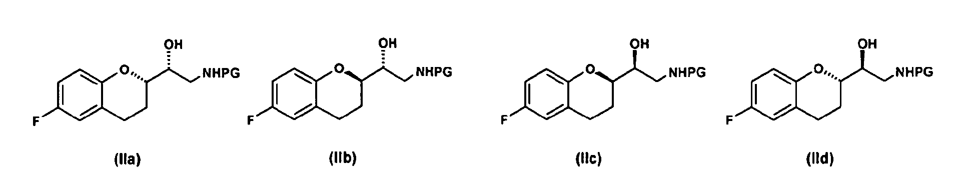 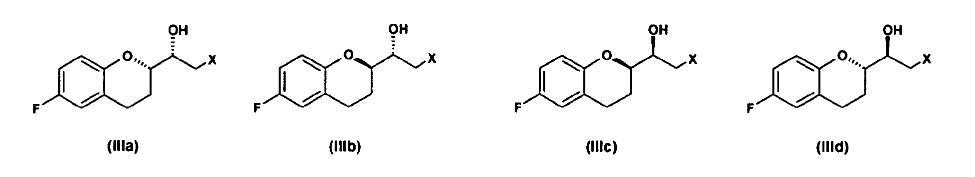 wobei durch Kopplung von (**IIa**) mit (**IIIb**) oder (**IIb**) mit (**IIIa**) d-Nebivolol (**Ia**) und durch Kopplung von (**IIc**) mit (**IIId**) oder (**IId**) mit (**IIIc**) I-Nebivolol (**Ib**) gewonnen wird und PG in den Intermediaten der Formel (**IIa-d**) ein Wasserstoffatom oder eine Aminschutzgruppe bedeutet und X in den Vorläufern der Formel (**IIIa-d**) ein Halogenatom, eine Hydroxylgruppe, eine Acylgruppe, eine Alkylsulfonyloxygrupe oder eine Arylsulfonyloxygruppe bedeutet,
die Intermediate der Formel (**IIa-d**) individuell aus den jeweiligen direkten Vorläufern der Formel (**IIIa-d**) gebildet werden, wobei Intermediat (**IIa**) aus (**IIIa**), Intermediat (**IIb**) aus (**IIIb**), Intermediat (**IIc**) aus (**IIIc**) und Intermediat (**IId**) aus (**IIId**) gebildet wird.

9. Verfahren gemäß Anspruch 8, **dadurch gekennzeichnet, dass** die Intermediate der Formel (**IIa-d**) aus den Vorläufern der Formel (**IIIa-d**) durch Umsetzung mit Ammoniak oder einem Arylamin, vorzugsweise Benzylamin, gebildet werden.

10. Verfahren zur Synthese von Enantiomeren des Nebivolol der Formel (**I**) 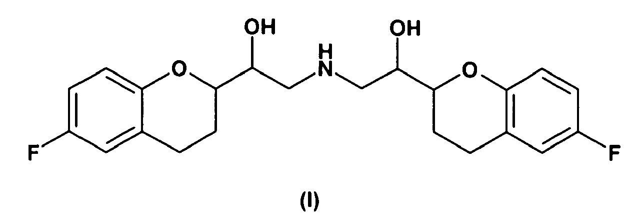 wobei die Enantiomere des Nebivolol durch individuelle Kopplungen der 4 enantiomerenreinen Intermediate der Formel (**IIa-d**) mit den korrespondierenden Vorläufern der Formel (**IIIa-d**) synthetisiert werden, 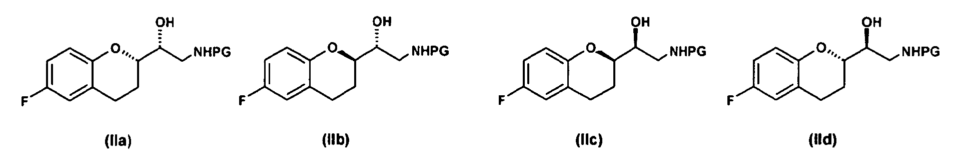 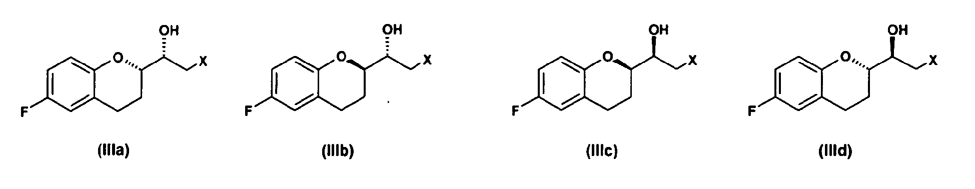 wobei PG in den Intermediaten der Formel (**IIa-d**) ein Wasserstoffatom oder eine Aminschutzgruppe bedeutet und X in den Vorläufern der Formel (**IIIa-d**) ein Halogenatom, eine Hydroxylgruppe, eine Acylgruppe, eine Alkylsulfonyloxygrupe oder eine Arylsulfonyloxygruppe bedeutet, und
die Intermediate der Formel (**IIa-d**) individuell aus den jeweiligen direkten Vorläufern der Formel (**IIIa-d**) gebildet werden, wobei Intermediat (**IIa**) aus (**IIIa**), Intermediat (**IIb**) aus (**IIIb**), Intermediat (**IIc**) aus (**IIIc**) und Intermediat (**IId**) aus (**III-d**) gebildet wird.

## Claims

1. Method for synthesis of racemic nebivolol represented by formula (**I**) 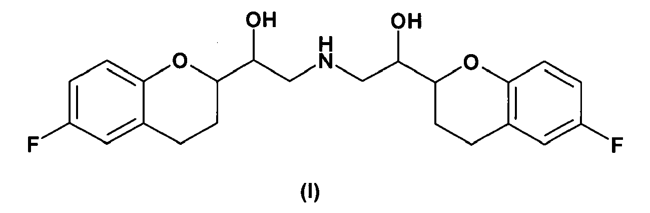 from the enantiomerically-pure compounds represented by formula IVa and IVb 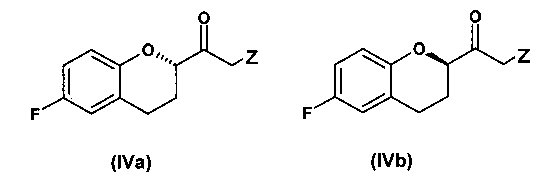 whereby racemic nebivolol is obtained through mixing the enantiomerically-pure compounds d-nebivolol (Ia) and l-nebivolol (Ib), 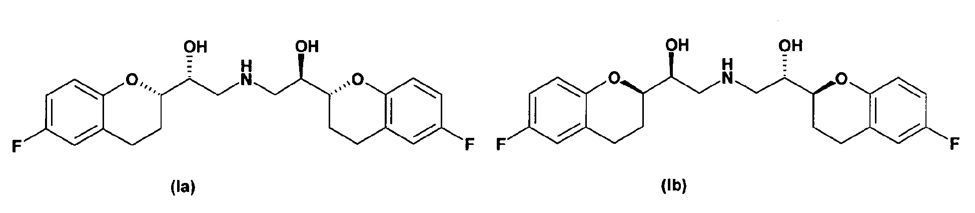 the compounds d-nebivolol (**Ia**) and l-nebivolol (**Ib**) are synthesised independent of each other as enantiomerically-pure compounds through producing (**Ia**) and (**Ib**) each through individual coupling of the **4** enantiomerically-pure intermediates represented by formula (**IIa-d**) to the corresponding precursors represented by formula (**IIIa-d**); 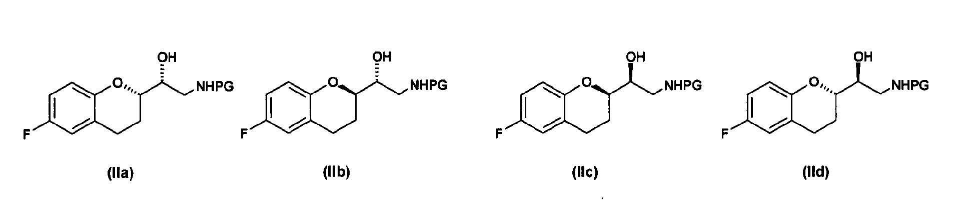 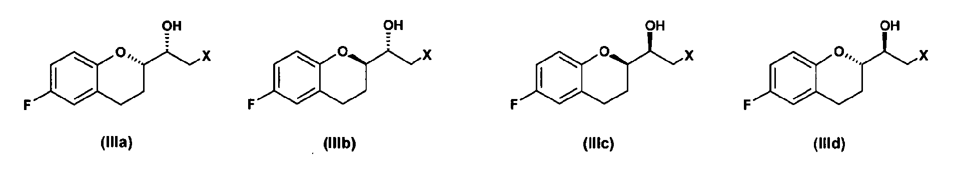 whereby d-nebivolol (**Ia**) is obtained through coupling (**IIa**) to (**IIIb**) or (**IIb**) to (**IIIa**) and l-nebivolol (**Ib**) is obtained through coupling (**IIc**) to (**IIId**) or (**IId**) to (**IIIc**), and PG in the intermediates represented by formula (**IIa-d**) is a hydrogen atom or an amine protection group, and X in the precursors represented by formula (**IIIa-d**) is a halogen atom, a hydroxyl group, an acyl group, an alkylsulfonyloxy group or an arylsulfonyloxy group,
the intermediates represented by formula (**IIa-d**) are formed individually from the respective direct precursors represented by formula (**IIIa-d**), whereby intermediate (**IIa**) is formed from (**IIIa**), intermediate (**IIb**) is formed from (**IIIb**), intermediate (**IIc**) is formed from (**IIIc**), and intermediate (**IId**) is formed from (**IIId**);
the precursors represented by formula (**IIIa-d**) are formed specifically through reduction of the enantiomerically-pure ketone precursors represented by formula (**IVa,b**), whereby the precursors represented by formula (**IIIa**) and (**III-d**) originate from the ketone precursor represented by formula (**IVa**), and the precursors represented by formula (**IIIb**) and (**IIIc**) originate from the ketone precursor represented by formula (**IVb**), and Z in the ketone precursors (**IVa,b**) is a halogen atom, a hydroxyl function, an acyl group, an alkylsulfonyloxy group or an arylsulfonyloxy group.

2. Method according to claim 1, **characterised in that** the intermediates (**IIIa-d**) are formed from the enantiomerically-pure ketone precursors (**IVa,b**) through diastereoselective reduction using a highly selective chiral reduction agent.

3. Method according to claim 2, **characterised in that** a reduction agent selected from chiral complex hydrides, chiral boron compounds, chiral catalysts for transfer hydrogenations and catalytic hydrogenations, and enzymes is used for reduction of compounds (**IVa,b**).

4. Method according to claim 3, **characterised in that** enzymes are used for reduction of compounds (**IVa,b**).

5. Method according to claim 4, **characterised in that** alcohol dehydrogenases and ketoreductases are used with or without cofactor regeneration for reduction of compounds (**IVa,b**).

6. Method according to at least one of the claims 1 to 5, **characterised in that** the intermediates represented by formula (**IIa-d**) are formed from the precursors represented by formula (**IIIa-d**) through reaction with ammonia or an arylamine, preferably benzylamine.

7. Method for synthesis of nebivolol enantiomers represented by formula (**I**) 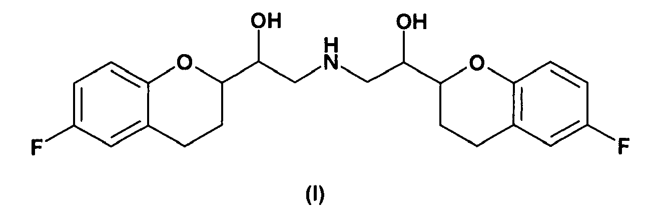 from the enantiomerically-pure compounds represented by formula IVa and IVb; 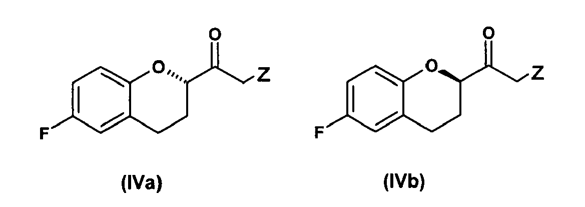 whereby the nebivolol enantiomers are synthesised through individual coupling of the 4 enantiomerically-pure intermediates represented by formula (**IIa-d**) to the corresponding precursors represented by formula (**IIIa-d**); 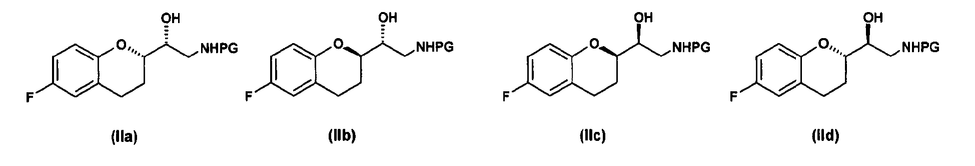 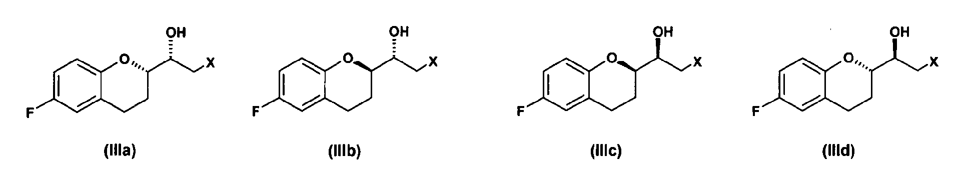 whereby PG in the intermediates represented by formula (**IIa-d**) is a hydrogen atom or an amine protection group, and X in the precursors represented by formula (**IIIa-d**) is a halogen atom, a hydroxyl group, an acyl group, an alkylsulfonyloxy group or an arylsulfonyloxy group;
the intermediates represented by formula (**IIIa-d**) are formed individually from the respective direct precursors represented by formula (**IIIa-d**), whereby intermediate (**IIa**) is formed from (**IIIa**), intermediate (**IIb**) is formed from (**IIIb**), intermediate (**IIc**) is formed from (**IIIc**), and intermediate (**IId**) is formed from (**IIId**);
the precursors represented by formula (**IIIa-d**) are formed specifically through reduction of the enantiomerically-pure ketone precursors represented by formula (**IVa,b**), whereby the precursors represented by formula (**IIIa**) and (**IIId**) originate from the ketone precursor represented by formula (**IVa**), and the precursors represented by formula (**IIIb**) and (**IIIc**) originate from the ketone precursor represented by formula (**IVb**), and Z in the ketone precursors (**IVa,b**) is a halogen atom, a hydroxyl function, an acyl group, an alkylsulfonyloxy group or an arylsulfonyloxy group.

8. Method for synthesis of racemic nebivolol represented by formula (**I**) 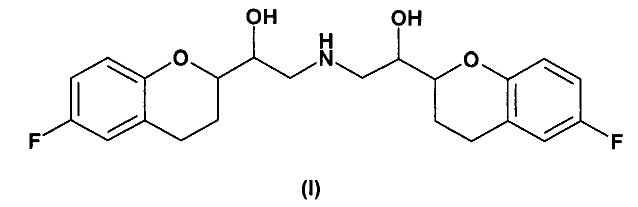 whereby racemic nebivolol is obtained through mixing the enantiomerically-pure compounds d-nebivolol (Ia) and l-nebivolol (Ib), 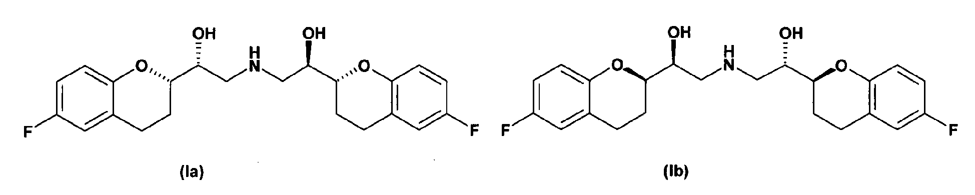 the compounds d-nebivolol (**Ia**) and l-nebivolol (**Ib**) are synthesised independent of each other as enantiomerically-pure compounds through producing (**Ia**) and (**Ib**) each through individual coupling of the 4 enantiomerically-pure intermediates represented by formula (**IIa-d**) to the corresponding precursors represented by formula (**IIIa-d**); 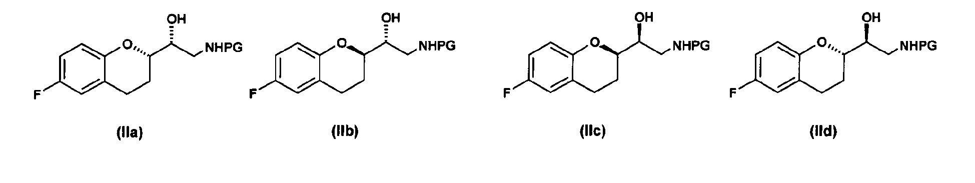 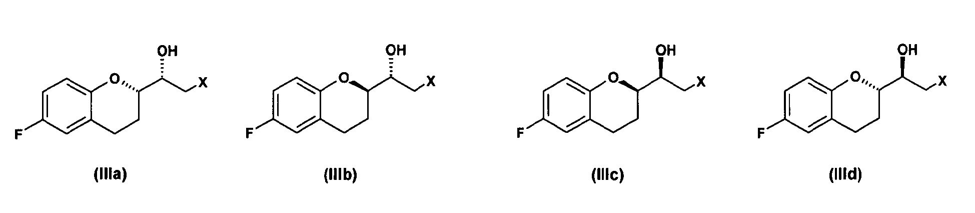 whereby d-nebivolol (**Ia**) is obtained through coupling (**IIa**) to (**IIIb**) or (**IIb**) to (**IIIa**) and l-nebivolol (**Ib**) is obtained through coupling (**IIc**) to (**IIId**) or (**IId**) to (**IIIc**), and PG in the intermediates represented by formula (**IIa-d**) is a hydrogen atom or an amine protection group, and X in the precursors represented by formula (**IIIa-d**) is a halogen atom, a hydroxyl group, an acyl group, an alkylsulfonyloxy group or an arylsulfonyloxy group,
the intermediates represented by formula (**IIa-d**) are formed individually from the respective direct precursors represented by formula (**IIIa-d**), whereby intermediate (**IIa**) is formed from (**IIIa**), intermediate (**IIb**) is formed from (**IIIb**), intermediate (**IIc**) is formed from (**IIIc**), and intermediate (**IId**) is formed from (**IIId**).

9. Method according to claim 8, **charcterised** in that the intermediates represented by formula (**IIa-d**) are formed from the precursors represented by formula (**IIIa-d**) through reaction with ammonia or an arylamine, preferably benzylamine.

10. Method for synthesis of nebivolol enantiomers represented by formula (I) 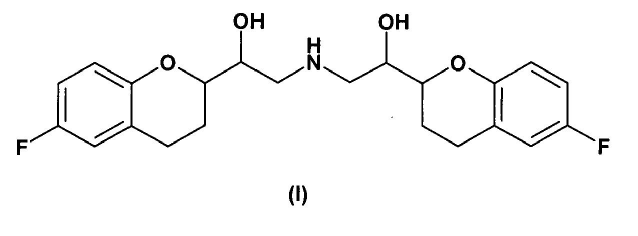 whereby the nebivolol enantiomers are synthesised through individual coupling of the 4 enantiomerically-pure intermediates represented by formula (**IIa-d**) to the corresponding precursors represented by formula (**IIIa-d**); 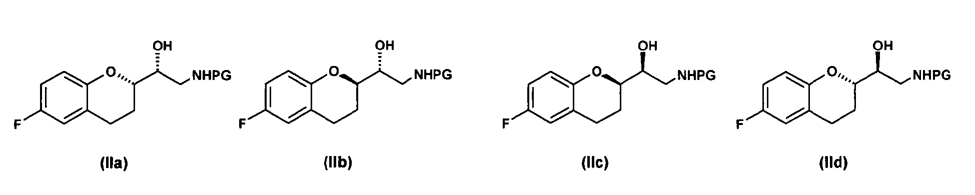 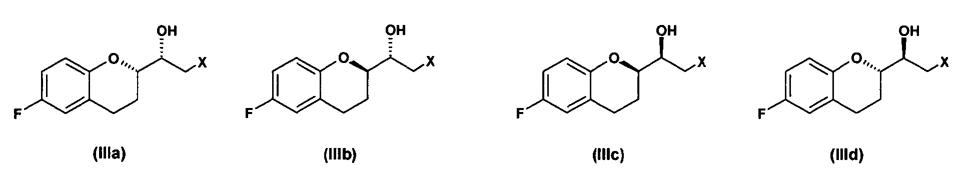 whereby PG in the intermediates represented by formula (IIa-d) is a hydrogen atom or an amine protection group, and X in the precursors represented by formula (IIIa-d) is a halogen atom, a hydroxyl group, an acyl group, an alkylsulfonyloxy group or an arylsulfonyloxy group;
the intermediates represented by formula (IIa-d) are formed individually from the respective direct precursors represented by formula (IIIa-d), whereby intermediate (IIa) is formed from (IIIa), intermediate (IIb) is formed from (IIIb), intermediate (IIc) is formed from (IIIc), and intermediate (IId) is formed from (IIId).

## Revendications

1. Procédé pour la synthèse de nébivolol racémique répondant à la formule (**I**) 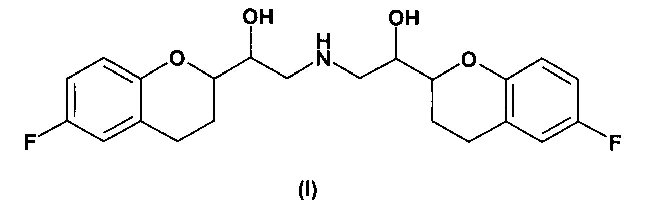 à partir des composés purs du point de vue énantiomère répondant aux formules IVa et IVb 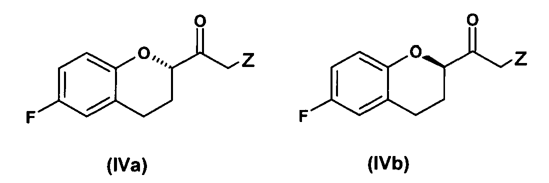 dans lequel on obtient le nébivolol racémique en mélangeant les composés purs du point de vue énantiomère d-nébivolol (**Ia**) et l-nébivolol (Ib), 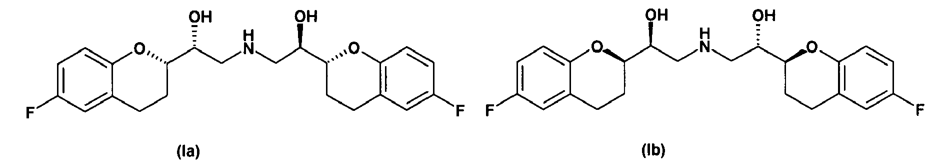 on synthétise les composés d-nébivolol (**Ia**) et l-nébivolol (**Ib**) indépendamment l'un de l'autre sous la forme de composés purs du point de vue énantiomère, en préparant (**Ia**) et (**Ib**) chaque fois via des couplages individuels des 4 produits intermédiaires purs du point de vue énantiomère répondant aux formules (**IIa-d**) avec les précurseurs correspondants répondant aux formules (**IIIa-d**), 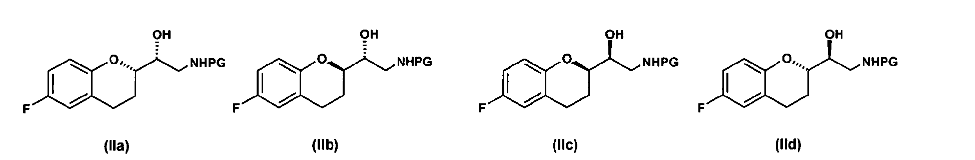 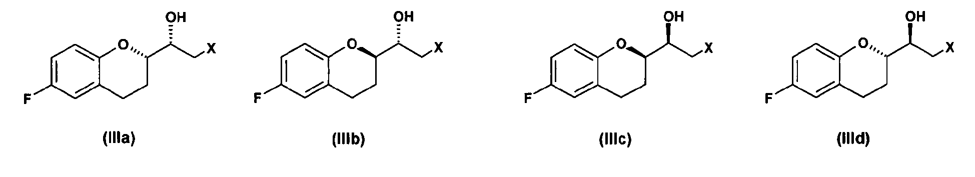 par lequel, via le couplage de (**IIa**) avec (**IIIb**) ou de (**IIb**) avec (**IIIa**), on obtient le d-nébivolol (**Ia**) et via le couplage de (**IIc**) avec (**IIId**) ou de (**IId**) avec (**IIIc**), on obtient le l-nébivolol (**Ib**) et dans lequel PG dans les produits intermédiaires répondant aux formules (**IIa-d**) représente un atome d'hydrogène ou un groupe de protection du groupe amine et X dans les précurseurs répondant aux formules (**IIIa-d**) représente un atome d'halogène, un groupe hydroxyle, un groupe acyle, un groupe alkylsulfonyloxy ou un groupe arylsulfonyloxy ;
on obtient les produits intermédiaires répondant aux formules (**IIIa-d**) de manière individuelle à partir des précurseurs directs respectifs répondant aux formules (**IIIa-d**), le produit intermédiaire (**IIa**) étant obtenu à partir de (**IIIa**), le produit intermédiaire (**IIb**) étant obtenu à partir de (**IIIb**), le produit intermédiaire (**IIc**) étant obtenu à partir de (**IIIc**) et le produit intermédiaire (**IId**) étant obtenu à partir de (**IIId**) ;
on obtient les précurseurs répondant aux formules (**IIIa-d**) de manière spécifique par réduction des substances de départ cétoniques pures du point de vue énantiomère répondant aux formules (**IVa,b**), les précurseurs répondant aux formules (**IIIa**) et (**IIId**) provenant de la substance de départ cétonique répondant à la formule (**IVa**), et les précurseurs répondant aux formules **(IIIb)** et (**IIIc**) provenant de la substance de départ cétonique répondant à la formule (**IVb**) et Z dans les substances de départ cétoniques (**IVa,b**) représentant un atome d'halogène, une fonction hydroxyle, un groupe acyle, un groupe alkylsulfonyloxy ou un groupe arylsulfonyloxy.

2. Procédé selon la revendication 1, **caractérisé en ce que** l'on obtient les précurseurs (**IIIa-d**) par réduction diastéréosélective avec un agent de réduction chiral très sélectif à partir des substances de départ cétoniques pures du point de vue énantiomère (**IVa,b**).

3. Procédé selon la revendication 2, **caractérisé en ce que**, pour la réduction des composés (**IVa,b**), on met en oeuvre un agent de réduction choisi parmi des hydrures complexes chiraux, des composés de bore chiraux, des catalyseurs chiraux pour des hydrogénations de transfert et des hydrogénations catalytiques, ainsi que des enzymes.

4. Procédé selon la revendication 3, **caractérisé en ce qu'**on met en oeuvre des enzymes pour la réduction des composés (**IVa,b**).

5. Procédé selon la revendication 4, **caractérisé en ce que**, pour les réductions des composés (**IVa,b**), on met en oeuvre des alcooldéshydrogénases et des cétoréductases avec ou sans régénération de cofacteurs.

6. Procédé selon au moins une des revendications 1 à 5, **caractérisé en ce qu'**on obtient les produits intermédiaires répondant aux formules (**IIa-d**) à partir des précurseurs répondant aux formules (**IIIa-d**) par mise en réaction avec de l'ammoniac ou avec une arylamine, de préférence une benzylamine.

7. Procédé pour la synthèse d'énantiomères du nébivolol répondant à la formule (**I**) 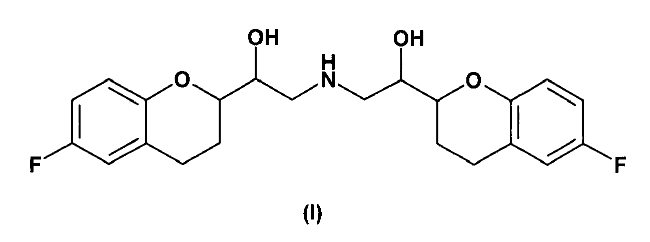 à partir des composés purs du point de vue énantiomère répondant aux formules IVa et IVb 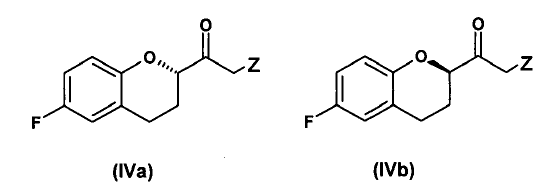 dans lequel on synthétise les énantiomères du nébivolol via des couplages individuels des 4 produits intermédiaires purs du point de vue énantiomère répondant aux formules (**IIa-d**) avec les précurseurs correspondants répondant aux formules (**IIIa-d**), 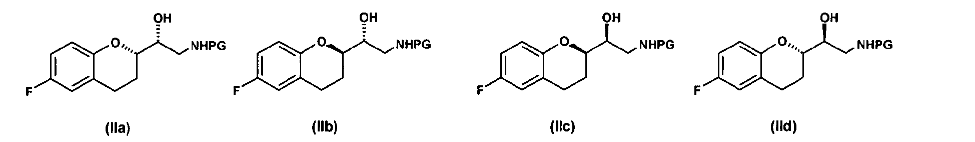 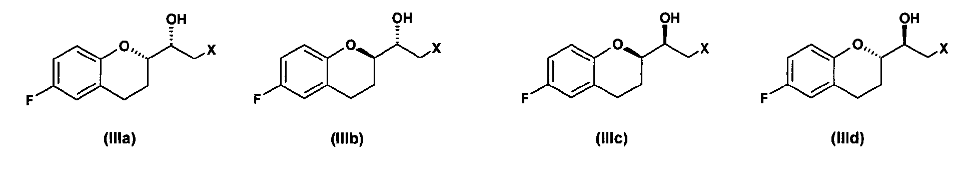 dans lequel PG dans les produits intermédiaires répondant aux formules (**IIa-d**) représente un atome d'hydrogène ou un groupe de protection du groupe amine et X dans les précurseurs répondant aux formules (**IIIa-d**) représente un atome d'halogène, un groupe hydroxyle, un groupe acyle, un groupe alkylsulfonyloxy ou un groupe arylsulfonyloxy ;
on obtient les produits intermédiaires répondant aux formules (**IIa-d**) de manière individuelle à partir des précurseurs directs respectifs répondant aux formules (**IIIa-d**), le produit intermédiaire (**IIa**) étant obtenu à partir de (**IIIa**), le produit intermédiaire (**IIb**) étant obtenu à partir de (**IIIb**), le produit intermédiaire (**IIc**) étant obtenu à partir de (**IIIc**) et le produit intermédiaire (**IId**) étant obtenu à partir de (**IIId**) ;
on obtient les précurseurs répondant aux formules (**IIIa-d**) de manière spécifique par réduction des substances de départ cétoniques pures du point de vue énantiomère répondant aux formules (**IVa,b**), les précurseurs répondant aux formules (**IIIa**) et (**IIId**) provenant de la substance de départ cétonique répondant à la formule (**IVa**), et les précurseurs répondant aux formules (**IIIb**) et (**IIIc**) provenant de la substance de départ cétonique répondant à la formule (**IVb**) et Z dans les substances de départ cétoniques (**IVa,b**) représentant un atome d'halogène, une fonction hydroxyle, un groupe acyle, un groupe alkylsulfonyloxy ou un groupe arylsulfonyloxy.

8. Procédé pour la synthèse de nébivolol racémique répondant à la formule (**I**) 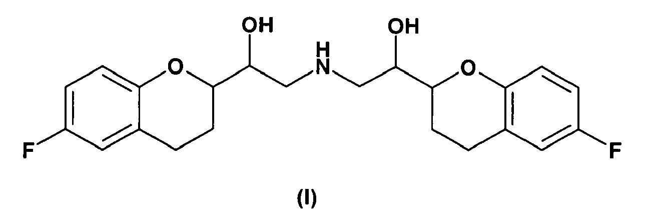 dans lequel on obtient le nébivolol racémique en mélangeant les composés purs du point de vue énantiomère d-nébivolol (la) et I-nébivolol (Ib), 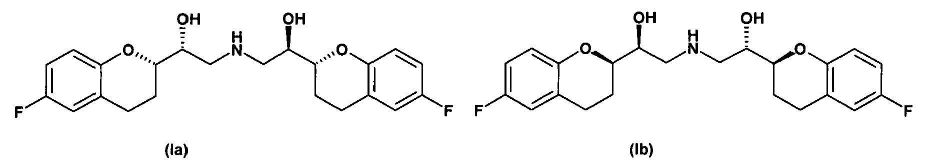 on synthétise les composés d-nébivolol (**Ia**) et I-nébivolol (**Ib**) indépendamment l'un de l'autre sous la forme de composés purs du point de vue énantiomère, en préparant (**Ia**) et (**Ib**) chaque fois via des couplages individuels des 4 produits intermédiaires purs du point de vue énantiomère répondant aux formules (**IIa-d**) avec les précurseurs correspondants répondant aux formules (**IIIa-d**), 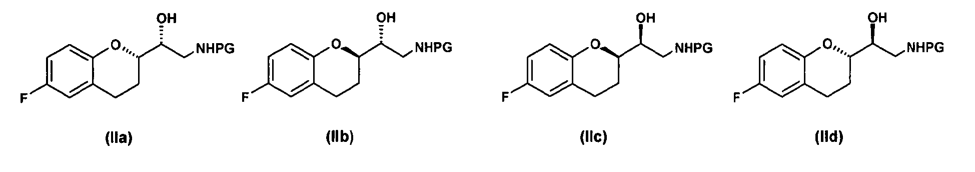 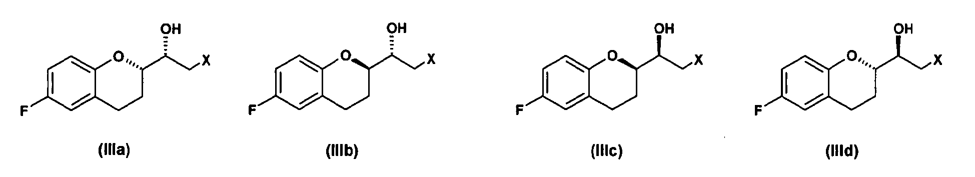 par lequel, via le couplage de (**IIa**) avec (**IIIb**) ou de (**IIb**) avec (**IIIa**), on obtient le d-nébivolol (**Ia**) et via le couplage de (**IIc**) avec (**IIId**) ou de (**IId**) avec (**IIIc**), on obtient le l-nébivolol (**Ib**) et dans lequel PG dans les produits intermédiaires répondant aux formules (**IIa-d**) représente un atome d'hydrogène ou un groupe de protection du groupe amine et X dans les précurseurs répondant aux formules (**IIIa-d**) représente un atome d'halogène, un groupe hydroxyle, un groupe acyle, un groupe alkylsulfonyloxy ou un groupe arylsulfonyloxy ;
on obtient les produits intermédiaires répondant aux formules (**IIa-d**) de manière individuelle à partir des précurseurs directs respectifs répondant aux formules (**IIIa-d**), le produit intermédiaire (**IIa**) étant obtenu à partir de (**IIIa**), le produit intermédiaire (**IIb**) étant obtenu à partir de (**IIIb**), le produit intermédiaire (**IIc**) étant obtenu à partir de (**IIIc**) et le produit intermédiaire (**IId**) étant obtenu à partir de (**IIId**).

9. Procédé selon la revendication 8, **caractérisé en ce qu'**on obtient les produits intermédiaires (**IIa-d**) à partir des précurseurs répondant aux formules (**IIIa-d**) par mise en réaction avec de l'ammoniac ou avec une arylamine, de préférence une benzylamine.

10. Procédé pour la synthèse d'énantiomères du nébivolol répondant à la formule (I) 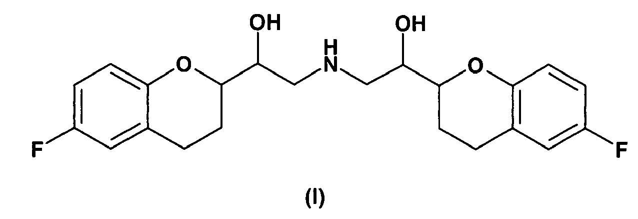 dans lequel on synthétise les énantiomères du nébivolol via des couplages individuels des 4 produits intermédiaires purs du point de vue énantiomère répondant aux formules (**IIa-d**) avec les précurseurs correspondants répondant aux formules (**IIIa-d**), 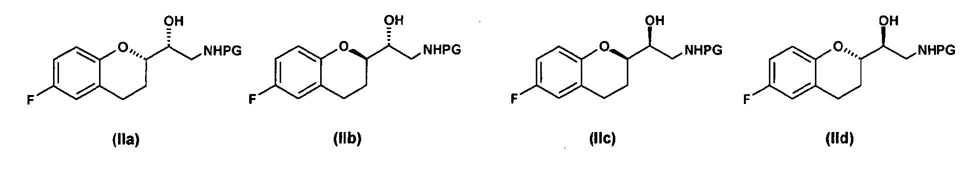 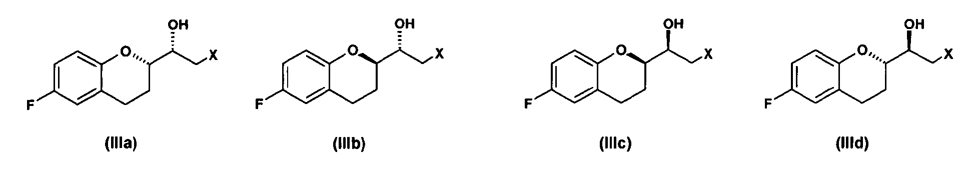 dans lequel PG dans les produits intermédiaires répondant aux formules (**IIa-d**) représente un atome d'hydrogène ou un groupe de protection du groupe amine et X dans les précurseurs répondant aux formules (**IIIa-d**) représente un atome d'halogène, un groupe hydroxyle, un groupe acyle, un groupe alkylsulfonyloxy ou un groupe arylsulfonyloxy ; et
on obtient les produits intermédiaires répondant aux formules (**IIa-d**) de manière individuelle à partir des précurseurs directs respectifs répondant aux formules (**IIIa-d**), le produit intermédiaire (**IIa**) étant obtenu à partir de (**IIIa**), le produit intermédiaire (**IIb**) étant obtenu à partir de (**IIIb**), le produit intermédiaire (**IIc**) étant obtenu à partir de (**IIIc**) et le produit intermédiaire (**IId**) étant obtenu à partir de (**IIId**).
